# FASCICULE DE BREVET EUROPEEN

(11) **EP 1 497 664 B1**
(45) Date de publication et mention de la délivrance du brevet: **21.03.2007**
(21) Numéro de dépôt: 03747160.4
(22) Date de dépôt: 24.04.2003
(51) Int. Cl.: G01N 33/72

(54) **PROCEDE POUR DETECTER ET LOCALISER DES TRACES DE SANG ET COMPOSE POUR DETECTER DES TRACES DE SANG**
VERFAHREN ZUM NACHWEIS UND ZUR LOKALISIERUNG VON BLUTSPUREN UND VERBINDUNG ZUM NACHWEIS VON BLUTSPUREN
METHOD OF DETECTING AND LOCATING TRACES OF BLOOD AND A COMPOUND FOR DETECTING TRACES OF BLOOD

(30) Priorité: 25.04.2002 FR 0205230
(43) Date de publication de la demande: 19.01.2005
(73) Titulaire: Roc Import, 98000 Monaco (MC)
(72) Inventeur: LEFEBVRE-DESPEAUX, Jean-Marc, 98000 Monaco (MC); BLUM, Loic, F-69300 Caluire (FR)
(74) Mandataire: Faber, Jean-Paul
(86) Numéro de dépôt international: PCT/FR2003/001299
(87) Numéro de publication internationale: WO 2003/091687

(56) Documents cités:
- WEBER K.: "Die Anwendung der Chemiluminescenz des Luminols in der gerichtilichen Medizin und Toxicologie. I. Der Nachweis von Blutspuren" DEUTSCHE ZEITSCHRIFT FÜR GERICHTLICHE MEDIZIN, vol. 57, 1966, pages 410-423, XP008015417 cité dans la demande
- PROESCHER F. ET AL.: "Detection of blood by means of chemiluminescence" JOURNAL OF LABORATORY CLINICAL MEDICINE, vol. 24, 1939, pages 1183-1189, XP008015575 cité dans la demande

## Description

L'invention concerne une composition pour détecter des traces de sang humain ou animal ainsi qu'un nécessaire et un procédé pour la préparation de cette composition. L'invention concerne également un procédé pour détecter et localiser des traces de sang humain ou animal.

La recherche et la localisation de traces de sang humain et animal par chimiluminescence est déjà connue.

La luminescence désigne l'ensemble des phénomènes d'émissions de rayonnements électromagnétiques ultraviolets, visibles ou infrarouges qui ne sont pas dus à un effet thermique.

Certaines molécules, les luminophores, ont la propriété, lorsqu'elles sont portées à un état excité d'émettre cette "lumière froide" en retournant à leur état fondamental. Le phénomène peut être provoqué de différentes façons et, selon la nature de la source d'énergie permettant à une molécule d'atteindre l'état excité, différents types de luminescence peuvent être définis.

La chimiluminescence est l'émission de lumière produite directement ou indirectement par une réaction chimique. En général, les réactions de chimiluminescence sont des processus d'oxydation.

Pour la détection de sang humain ou animal, le luminol (5-amino-2,3-dihydro-1,4-phthalazinedione) est le composé chimiluminescent le plus utilisé bien que d'autres composés de luminol aient également été utilisés.

En solution aqueuse, la réaction de chimiluminescence du luminol requiert la présence d'un système d'oxydation et d'un environnement alcalin (White E.H. & Roswell, D.F. 1985. Luminol chemiluminescence. *Chemi-and Bioluminescence* (Burr, J.G., ed.), Marcel Dekker, New York, pp.215-244).

L'oxydation du luminol conduit à la formation de l'ion aminophthalate à l'état excité, dont le retour à l'état fondamental s'accompagne d'une émission de lumière.

Le rendement quantique de la réaction est faible (environ 0,01) et le spectre d'émission présente un maximum à 430 nm (couleur bleu pâle) (White, E.H. & Roswell, D.F. 1985. *Luminol chemiluminescence. Chemi-and Bioluminescence (Burr, J.G., ed.), Marcel Dekker, New York, pp. 215-244).*

Les principaux composés pouvant catalyser cette réaction d'émission de lumière sont les métaux de transition (Cr³⁺, Mn⁴⁺, Fe²⁺, Fe³⁺, CO²⁺, Ni²⁺, Cu²⁺, Hg²⁺), libres ou complexés, l'hème et la peroxydase (enzyme extraite entre autre du raifort).

L'hème est une structure biochimique particulière qui fait partie intégrante de la peroxydase. Cette structure est également présente dans l'hémoglobine qui est la protéine de transport de l'oxygène et d'une partie du CO₂ dans le sang.

Ainsi, la présence d'hémoglobine, donc de sang, peut être mise en évidence en exploitant l'aptitude de l'hème à catalyser la chimiluminescence du luminol.

En d'autres termes, un mélange luminol/agent oxydant/agent alcalin mis au contact de sang émettra de la lumière.

Une méthode de détection de sang par une telle composition a donc été suggérée, il y a plus de 60 ans, comme test présomptif de la présence de sang dans le cadre d'enquêtes criminelles.

C'est ainsi que Specht, W. en 1937 dans *The Chemiluminescence of hemin: an aid for finding and recognizing blood stains important for forensic purposes. Angewande Chemie, 10, 155-157,* a le premier proposé de mettre en évidence la présence de traces de sang judiciairement importantes, sur la scène d'un crime grâce à une composition contenant 0,1 partie en poids de luminol, 3 parties en poids d'un agent alcalin qui est le carbonate de sodium et 15 parties en poids d'un agent oxydant qui est le peroxyde d'hydrogène à 30 % dilués dans 100 parties en poids d'eau distillée.

Specht, W. en 1937 dans *The Chemiluminescence of hemin: an aid for finding and recognizing blood stains important for forensic purposes. Angewande Chemie, 10, 155-157,* a étudié et démontré la sensibilité de la réaction de luminescence avec ce réactif sur des traces de sang séché puis dilué à 1:2 000 en volume.

Proescher, F. & Moody, A.M. en 1939 dans *Detection of blood by means of chemiluminescence. Journal of Laboratory Clinical Medicine., 24*, *1183-1189,* ont rappelé que Specht, W. en 1937 dans *The Chemiluminescence of hemin: an aid for finding and recognizing blood stains important for forensic purposes. Angewande Chemie, 10, 155-157,* avait fait des essais sur des broussailles, du sang exposé au soleil et à la pluie et que dans ces différentes conditions, la réaction de chimiluminescence se produisait.

Quant à eux, ils ont testé la composition de Specht, W. en 1937 dans *The Chemiluminescence of hemin: an aid for finding and recognizing blood stains important for forensic purposes. Angewande Chemie, 10, 155-157,* sur du sang animal aussi bien qu'humain jusqu'à des dilutions de 1:1 000 000 et ont démontré que la réaction de chimiluminescence se produisait dans les deux cas.

En 1951, Grodsky, M., Wright, K. & Kirk, P.L. dans *Simplified preliminary blood testing. An improved technique and comparative study of methods. Journal of the America Institute of Criminal Law and Criminalogy, 42, 95-104,* constatant que la sensibilité et la réactivité du luminol sont très difficilement contrôlables lorsque le peroxyde d'hydrogène est utilisé en tant qu'agent oxydant, a proposé d'utiliser un agent oxydant perborate. Il a également proposé un nécessaire de terrain constitué de différents récipients contenant chacun indépendamment le luminol, l'agent oxydant perborate et l'agent alcalin carbonate de sodium. Ce nécessaire contenait également des accessoires tels que des vaporisateurs en plastique et en verre, du papier filtre, une bouteille d'eau distillée, une lampe de poche pour permettre le mélange des réactifs dans le noir, et des récipients en plastique pour stocker et transporter les traces de sang ou d'autres preuves de petites tailles.

Là encore, Grodsky, M., Wright, K. & Kirk, P.L. en 1951 dans *Simplified preliminary blood testing. An improved technique and comparative study of methods. Journal of the America Institute of Criminal Law and Criminalogy, 42, 95-104,* s'est attaché à optimiser la réaction de chimiluminescence du luminol, en présence de sang très dilué, à savoir à une dilution de 1:5 000 000 en volume.

Proescher, F. & Moody, A.M. en 1939 dans *Detection of blood by means of chemiluminescence. Journal of Laboratory Clinical Medicine., 24, 1183-1189,* tout comme Grodsky , M., Wright, K. & Kirk, P.L. en 1951 dans *Simplified preliminary blood testing. An improved technique and comparative study of methods. Journal of the America Institute of Criminal Law and Criminalogy, 42, 95104,* recommandaient de d'abord vaporiser les lieux avec de l'acide chlorhydrique pour décomposer l'hémoglobine et augmenter la sensibilité du test.

Weber, K. en 1966 dans *Die Anweldung der Chemiluminescenz des Luminols in der Gerichtlichen Medizin und Toxicologie. I. Der Nachweis von Blutspuren. Deutsche Zeitschrift für die gesamte Gerichtliche Medizin, 57, 410-423*, a mis en évidence le fait que l'utilisation d'un agent alcalin carbonate ne provoquait qu'une réaction lente d'oxydation de l'hémoglobine et que, par conséquent, la luminescence était beaucoup plus faible en utilisant un tel agent alcalin carbonate. Il préconisait donc l'emploi d'hydroxyde de sodium en tant qu'agent alcalin. Weber, K. en 1966 dans *Die Anweldung der Chemiluminescenz des Luminols in der Gerichtlichen Medizin und Toxicologie. I. Der Nachweis von Blutspuren. Deutsche Zeitschrift für die gesamte Gerichtliche Medizin, 57, 410-423,* a également constaté que les concentrations en luminol et en peroxyde d'hydrogène utilisées dans les réactifs de Specht, W. en 1937 dans *The Chemiluminescence of hemin: an aid for finding and recognizing blood stains important for forensic purposes Angewande Chemie, 10, 155-157,* étaient trop fortes et provoquaient une inhibition de concentration de la réaction du luminol liée à la concentration des réactifs et donc une disparition de la luminescence chimique et une baisse sensible dans la mise en évidence de traces de sang. Weber, K. en 1966 dans *Die Anweldung der Chemiluminescenz des Luminols in der Gerichtlichen Medizin und Toxicologie. L Der Nachweis von Blutspuren. Deutsche Zeitschrift für die gesamte Gerichtliche Medizin, 57, 410-423,* a donc proposé une composition contenant 0,4 mmoles/L de luminol, 17,6 mmoles/L de peroxyde d'hydrogène et 45 mmoles/L d'hydroxyde de sodium ou d'hydroxyde de potassium, dilués dans de l'eau distillée, pour détecter des traces de sang séché ou frais jusqu'à une dilution de 1:20 000 000.

En 1990, Grispino, R.R.J. dans *The effects of luminol on serological analysis of dried blood stains Crime Laboratory Digest, 17(1), 13-23,* a proposé d'utiliser une composition de luminol contenant 5,6 mmoles/L de luminol, 472 mmoles/L de carbonate de potassium ou sodium et 100 mmoles/L de peroxyde d'hydrogène H₂O₂, dilués dans de l'eau distillée, pour détecter des traces de sang séché plusieurs jours et de sang frais jusqu'à des dilutions de ce sang de 1:10 000 à 1:100 000.

Ainsi, l'utilisation, pour détecter des traces de sang sur la scène d'un crime, d'une composition contenant du luminol en combinaison avec un agent alcalin et un agent oxydant, est connue de longue date avec l'inconvénient que le sang ayant subi la détection ne se prête plus à une analyse des groupes sanguins.

Pour cette application, il a été préconisé d'employer en tant qu'agent oxydant du peroxyde d'hydrogène plutôt que du perborate de sodium car le perborate de sodium est peu soluble dans l'eau et bouche le dispositif de vaporisation de la composition. Il a également été préconisé d'utiliser en tant qu'agent alcalin de l'hydroxyde de sodium ou de l'hydroxyde de potassium plutôt qu'un composé de carbonate car la réaction d'oxydation du luminol était plus lente avec un composé de carbonate qu'avec un composé hydroxyde et donc l'émission de lumière était moins intense avec un composé de carbonate.

Weber, K. en 1966 dans *Die Anweldung der Chemiluminescenz des Luminols in der Gerichtlichen Medizin und Toxicologie. I. Der Nachweis von Blutspuren. Deutsche Zeitschrift für die gesamte Gerichtliche Medizin, 57, 410-423,* a alors proposé une composition de ce type mais contenant de faibles quantités de luminol et de peroxyde d'hydrogène car des quantités trop fortes de ces composés entraînent une inhibition de la réaction du luminol et donc une disparition de la luminescence chimique et une baisse sensible dans la mise en évidence de traces de sang.

Par ailleurs, Byrne, dans les brevets U.S. 5 770 116 et 5 833 887, a proposé d'utiliser la réaction de chimiluminescence du luminol pour détecter les traces de sang perdu par un gibier blessé, sur un terrain de chasse, dans des conditions de lumière réduite.

A cet effet, Byrne, dans les brevets U.S. 5 770 116 et 5 833 887, préconise l'emploi d'une composition contenant, outre le luminol, du perborate de sodium en tant qu'oxydant et du carbonate de sodium en tant qu'agent alcalin.

Cependant, cette composition présente les inconvénients liés à l'utilisation du perborate de sodium et du carbonate de sodium précédemment décrits.

Cela est particulièrement gênant, pour l'application à la chasse, en particulier lorsque la détection s'effectue non pas en pleine nuit mais dans des conditions simplement de lumière réduite, comme à la tombée de la nuit, où la perte d'intensité lumineuse émise par la réaction du luminol avec le sang est particulièrement gênante car cette intensité lumineuse pourrait ne pas être suffisante pour permettre de détecter les traces de sang.

De plus, toutes les études de l'art antérieur ont été effectuées sur du sang très dilué.

L'invention vise à pallier les inconvénients des compositions de l'art antérieur en proposant une composition utilisable dans les conditions réelles d'utilisation, c'est-à-dire qui permet de révéler des traces mêmes infimes de sang pur, instantanément et avec une intensité lumineuse suffisante pour être visibles, non seulement dans une obscurité totale mais également dans des conditions de lumière seulement réduite qui correspondent à celles de la tombée du jour.

A cet effet, l'invention propose une composition pour la détection de traces de sang humain ou animal du type comprenant un composé de luminol, un agent oxydant et une base, dilués dans un solvant de préférence aqueux, caractérisée en ce que le composé de luminol est présent en une quantité fournissant une concentration de 1 à 20 mmoles/L dans la composition finale, l'agent oxydant est du peroxyde d'hydrogène qui est présent en une quantité fournissant une concentration de 25 à 100 mmoles/L dans la composition finale, et la base est de la soude NaOH qui est présente en une quantité fournissant une concentration comprise entre 25 mmoles/L et 500 mmoles/L dans la composition finale.

Selon un mode de réalisation avantageux, le composé de luminol est présent en une quantité fournissant une concentration comprise entre 1 et 10, mieux est de l'ordre de 5 mmoles/L .

Par ailleurs, la quantité en soude NaOH sera pour de nombreuses applications avantageusement limitée à 25 à 150 mmoles/L.

Il est préféré que le pH de la composition soit inférieur à environ 11,5, comme cela sera expliqué plus loin.

Il est bien compréhensible à l'homme de l'art que la concentration de chaque composant est réglée en fonction de la concentration des autres composants.

Egalement, l'expression "composé de luminol" utilisée dans la description et les revendications signifie le luminol et tout composé précurseur ou dérivé du luminol par exemple formé par substitution du luminol avec au moins un substituant tel que alkyl, notamment méthyl, amine, hydroxy, etc et capable de fournir une chimiluminescence du type de celle du luminol. Des exemples de tels composés sont le diéthyl isoluminol et l'aminobutyléthyl isoluminol.

De préférence, le composé de luminol est le luminol.

Selon un premier mode de réalisation préféré, la soude NaOH est présente à une concentration d'environ 90 mmoles/L dans la composition finale.

Selon un second mode de réalisation préféré, la soude NaOH est présente à une concentration de 25 à 50 mmoles/L dans la composition finale.

Dans tous les cas, de préférence, le solvant aqueux est de l'eau, encore mieux de l'eau non gazeuse.

Un autre objet de l'invention est l'utilisation de la composition de l'invention pour détecter des traces de sang humain ou animal.

La composition selon le premier mode de réalisation est particulièrement appropriée pour détecter des traces de sang animal sur un terrain de chasse.

La composition selon le second mode de réalisation est particulièrement appropriée pour détecter des traces de sang humain sur la scène d'un crime ou d'un accident.

L'invention propose également un nécessaire pour la préparation de la composition de l'invention qui dans une première forme préférée comprend :
- dans un premier récipient au moins une dose individuelle contenant un composé de luminol en une quantité fournissant de 1 à 20 mmoles,
- dans un second récipient au moins une dose individuelle contenant de 25 à 100 mmoles de peroxyde d'hydrogène, et
- dans un troisième récipient au moins une dose individuelle contenant de 25 à 500 mmoles de soude NaOH.

Dans une première variante, cette première forme préférée du nécessaire de l'invention est adaptée pour la préparation de la composition de l'invention selon le premier mode de réalisation, et comprend :
- dans un premier récipient au moins une dose individuelle contenant ledit composé de luminol, en particulier en une quantité fournissant de 1 à 10 mmoles,
- dans un second récipient au moins une dose individuelle contenant de 25 à 100 mmoles de peroxyde d'hydrogène, et
- dans un troisième récipient au moins une dose individuelle contenant de 25 à 150 mmoles de soude NaOH.

Dans une seconde variante, cette première forme préférée du nécessaire de l'invention est adaptée pour la préparation de la composition de l'invention selon le second mode de réalisation, en particulier pour une utilisation sur le lieu d'un crime, et comprend :
- dans un premier récipient au moins une dose individuelle contenant un composé de luminol en une quantité fournissant environ 5 mmoles,
- dans un second récipient au moins une dose individuelle contenant environ 50 mmoles de peroxyde d'hydrogène, et
- dans un troisième récipient au moins une dose individuelle contenant de 25 à 50 mmoles de NaOH.

Dans une deuxième forme préférée, le nécessaire pour la préparation de la composition selon l'invention comprend :
- dans un premier récipient au moins une dose individuelle contenant ledit composé de luminol en une quantité fournissant de 1 à 20 mmoles en pré mélange avec soit de la soude NaOH en une quantité comprise entre 25 et 500 mmoles, soit avec de 25 à 100 mmoles de peroxyde d'hydrogène sous forme compatible solide,
- dans un second récipient au moins une dose individuelle contenant de 25 à 100 mmoles de peroxyde d'hydrogène, ou de 25 à 500 mmoles de soude, selon le pré mélange du premier récipient.

Avantageusement, ce nécessaire pour la préparation d'une composition selon l'invention comprend :
- dans un premier récipient au moins une dose individuelle contenant un composé de luminol en une quantité fournissant de 1 à 20 mmoles de composé de luminol en pré mélange avec soit de 25 à 150 mmoles de soude NaOH, soit de 25 à 100 mmoles de peroxyde d'hydrogène, sous forme compatible solide, et
- dans un second récipient au moins une dose individuelle contenant 50 mmoles de peroxyde d'hydrogène, ou de 25 à 150 mmoles de soude NaOH, selon le pré mélange du premier récipient.

Il est à noter que le peroxyde d'hydrogène sous forme solide est disponible dans le commerce, en particulier auprès de SIGMA-ALDRICH, en pastilles ou en poudre, sous forme de produit d'addition avec l'urée, dit "Urea Adduct".

Alternativement, ce nécessaire comprend :
- dans un premier récipient au moins une dose individuelle contenant un composé de luminol en une quantité fournissant 5 mmoles de luminol en pré mélange, soit avec de 25 à 50 mmoles, ou 90 mmoles, de soude NaOH, soit avec 50 mmoles de peroxyde d'hydrogène sous forme compatible solide, et
- dans un second récipient au moins une dose individuelle contenant soit 50 mmoles de peroxyde d'hydrogène, soit de 25 à 50 mmoles, ou 90 mmoles, de soude NaOH, en fonction du pré mélange.

Selon encore un autre mode avantageux de l'invention, les trois composants principaux de l'invention peuvent être formulés en pré mélange unique dans des formulations bien connues de l'homme de l'art de la formulation, permettant leur compatibilité sans réaction prématurée de façon à ce qu'ils soient inclus dans un récipient unique. Ainsi, il est bien connu certaines techniques d'enrobage évitant les réactions prématurées. L'invention concerne ainsi également une formulation combinée des trois composants de base de l'invention pré mélangés dans un récipient unique.

Dans tous les cas, dans le nécessaire selon l'invention, chaque récipient est de préférence un récipient refermable en matière plastique ou en verre.

Ces récipients peuvent aussi être des sachets contenant les doses individuelles sous forme de poudre.

Plus préférablement, au moins un ou chaque récipient est formé par une alvéole conformée dans au moins un blister.

Encore plus préférablement, le nécessaire selon l'invention comprend au moins un blister à au moins trois alvéoles, l'une de ces au moins trois alvéoles contenant une dose individuelle contenant un composé de luminol en une quantité fournissant de 1 à 20 mmoles, une autre de ces au moins trois alvéoles contenant une dose individuelle contenant de 25 à 500 mmoles de soude NaOH et encore une autre de ces au moins trois alvéoles contenant de 25 à 100 mmoles de peroxyde d'hydrogène.

Alternativement, il comprend au moins un blister à au moins deux alvéoles, l'une de ces au moins deux alvéoles contenant une dose individuelle contenant un composé de luminol en une quantité fournissant de 1 à 20 mmoles en pré mélange avec soit de la soude NaOH en une quantité de 25 à 500 mmoles, soit avec de 25 à 100 mmoles de peroxyde d'hydrogène sous forme compatible et solide, et l'autre de ces au moins deux alvéoles contenant une dose individuelle contenant soit de 25 à 100 mmoles de peroxyde d'hydrogène, soit de 25 à 500 mmoles de soude, selon le pré mélange de la première alvéole.

Selon une troisième variante, le nécessaire comprend au moins un blister à au moins une alvéole qui contient une dose individuelle d'une formulation pré mélangée sous forme compatible solide des trois composants de base de l'invention.

De préférence, dans toutes les formes variantes du nécessaire selon l'invention l'une au moins des doses individuelles est sous la forme de pastille.

Plus avantageusement, dans le nécessaire selon l'invention, chaque dose de luminol, de peroxyde d'hydrogène, et de soude est sous la forme de pastille.

Dans tous les cas, avantageusement, chaque dose individuelle contient de plus des excipients facilitant la compression directe et la désagrégation de la pastille.

L'invention propose encore un procédé de reconstitution de la composition selon l'invention, caractérisé en ce qu'il consiste à diluer dans de l'eau une dose individuelle de composé de luminol, une dose individuelle de peroxyde d'hydrogène et une dose individuelle de soude, chaque dose individuelle étant prélevée dans les récipients du nécessaire selon l'invention.

L'invention propose aussi un procédé de recherche et de localisation, dans des conditions de lumière réduite ou d'absence totale de lumière, d'un animal blessé ou abattu, caractérisé en ce qu'on pulvérise la composition selon l'invention ou la composition obtenue selon le procédé de reconstitution de l'invention, sur les zones du terrain où l'on soupçonne l'animal d'être passé, pour produire une réaction lumineuse par contact de la composition avec les traces de sang perdu par l'animal.

Toujours un autre objet de l'invention est un procédé de recherche et de localisation de traces de sang humain sur la scène de crime, caractérisé en ce qu'on pulvérise, dans des conditions de lumière réduite ou d'absence totale, la composition selon l'invention ou la composition obtenue par le procédé de reconstitution selon l'invention, sur cette scène d'un crime, pour produire une réaction lumineuse par contact de la composition avec les traces de sang humain.

L'invention sera mieux comprise et d'autres buts, détails et avantages de celle-ci apparaîtront mieux à la lecture de la description explicative qui va suivre.

Selon l'invention, on a découvert d'une manière surprenante qu'une composition comprenant de 1 à 20 mmoles/L de composé de luminol, de 25 à 100 mmoles/L de peroxyde d'hydrogène (H₂O₂) en tant qu'agent oxydant, et entre 25 mmoles/L et 500 mmoles/L de soude (NaOH) en tant qu'agent alcalin, dilués dans un solvant de préférence aqueux, permet de détecter rapidement avec efficacité des traces de sang humain ou animal, frais ou séché, lavé ou non.

Dans cette composition, il est remarquable qu'une concentration en composé de luminol de 1 à 20 mmoles, de préférence de 1 à 10 mmoles/L permet d'obtenir l'intensité lumineuse optimale, sans utiliser de fortes quantités de composé de luminol.

En effet, en présence de 25 à 500 mmoles, avantageusement de 25 à 150 mmoles, mieux environ 90 mmoles/L de composition finale d'hydroxyde de sodium NaOH et de 25 à 100 mmoles/L de peroxyde d'hydrogène, H₂O₂, l'intensité lumineuse augmente avec la concentration en composé de luminol, mais atteint pratiquement un plateau à partir de 10 à 20 mmoles/L de composé de luminol. Cependant, selon l'invention, il est inattendu qu'avec une concentration faible, en particulier d'environ 5 mmoles/L en composé de luminol, 93 % de l'intensité lumineuse maximale soit obtenue.

Le choix de l'agent oxydant s'est porté sur le peroxyde d'hydrogène, H₂O₂, non seulement en raison de sa grande disponibilité à faible coût mais également car, comme relevé par Grispino, R.R.J. dans *The effects of luminol on serological analysis of dried blood stains Crime Laboratory Digest, 17(1), 13-23,* l'utilisation d'un agent oxydant perborate, peu soluble dans l'eau ou un solvant aqueux, entraîne un bouchage du dispositif de vaporisation de la solution et n'est donc pas pratique d'emploi.

De plus, il a pu être observé de manière surprenante que c'est avec le peroxyde d'hydrogène que l'intensité lumineuse la plus élevée est obtenue.

Pour mieux faire comprendre l'invention, on va décrire maintenant, plusieurs modes de mise en oeuvre, à titre d'exemples purement illustratifs et non limitatifs. Les exemples 1 à 4 font partie de l'invention et sont revendiqués en tant que tels. D'autres exemples de l'invention fournissant des résultats équivalents sont :
- une composition comprenant de 10 à 20 mmoles/L de composé de luminol, environ 40 mmoles de NaOH et environ 50 mmoles de H₂O₂.
- une composition comprenant environ 5 mmoles/L de composé de luminol, 50 mmoles/L de H₂O₂ et environ 120 mmoles/L de NaOH.

### Exemple 1 selon l'invention

On a préparé une composition selon l'invention contenant 5 mmoles/L de luminol, 50 mmoles/L de NaOH et 50 mmoles/L de H₂O₂, dilués dans 1 litre d'eau distillée.
Cette composition a été vaporisée sur :
- du sang de mouton dilué à 1:1 000 000, sur du sang de mouton dilué à 1:100 000,
- du sang de mouton pur frais, et
- du sang de mouton pur séché.

Le pH de la composition a été mesuré. Les intensités lumineuses obtenues lors des réactions entre la composition de l'invention et chacun des différents échantillons de sang ont été mesurées selon les protocoles suivants.

En ce qui concerne le sang dilué, la mesure d'intensité lumineuse a été réalisée à l'aide d'un luminomètre LUMAC, modèle Biocounter M2500 fabriqué et commercialisé par la Société LUMAC, cet appareil étant modifié pour être équipé d'un tube photomultiplicateur HAMAMATSU, et couplé à son enregistreur qui enregistre l'intensité lumineuse en unité arbitraire, selon la méthode habituelle des spécialistes en luminométrie.

En ce qui concerne la mesure d'intensité lumineuse sur le sang pur frais ou le sang pur séché, il est utilisé une caméra CCD de marque FUJIFILM, modèle LAS-1000, couplée à un ordinateur équipé d'un logiciel de traitement et fournissant le pic d'intensité ainsi que la durée de réaction de manière automatisée.

Les durées de réaction entre la composition de l'invention et chacun des différents échantillons de sang ont été mesurées selon le protocole suivant.

On estime que la réaction est complète en suivant la courbe d'intensité lumineuse enregistrée pour le sang dilué par l'enregistreur couplé au luminomètre lorsque le pic de maximum d'intensité lumineuse est atteint ; ou bien on prend la valeur donnée par l'ordinateur dans le cas du sang pur. Comme on pourra l'observer à partir du tableau 1, dans le cas du présent exemple 1 selon l'invention, sur le sang pur frais et le sang pur séché, le pic maximum d'intensité lumineuse est obtenu 40 secondes après l'instant de vaporisation de la composition.

Les résultats de ces mesures sont regroupés au tableau 1 ci-après.

### Exemple 2 selon l'invention

On a préparé une composition selon l'invention contenant 5 mmoles/L de luminol, 90 mmoles/L de NaOH et 50 mmoles/L de H₂O₂, dilués dans 1 litre d'eau distillée.

Les mêmes mesures qu'à l'exemple 1 ont été effectuées, selon les mêmes protocoles.

Les résultats sont regroupés au tableau 1 ci-après.

### Exemple 3 selon l'invention

On a préparé une composition selon l'invention contenant 5 mmoles/L de luminol, 25 mmoles/L de NaOH et 50 mmoles/L de H₂O₂, dilués dans 1 litre d'eau distillée.

Les mêmes mesures qu'à l'exemple 1 ont été effectuées, selon les mêmes protocoles.

Les résultats sont regroupés au tableau 1 ci-après.

### Exemple 4 selon l'invention

On a préparé une composition selon l'invention contenant 5 mmoles/L de luminol, 40 mmoles/L de NaOH et 50 mmoles/L de H₂O₂, dilués dans 1 litre d'eau distillée.

Les mêmes mesures qu'à l'exemple 1 ont été effectuées, selon les mêmes protocoles.

Les résultats sont regroupés au tableau 1 ci-après.

### Exemple comparatif 1

A titre de comparaison, on a préparé une composition contenant 5 mmoles/L de luminol, 10 mmoles/L de NaOH et 50 mmoles/L de H₂O₂, dilués dans un litre d'eau distillée.

Les mêmes mesures qu'à l'exemple 1 ont été effectuées, selon les mêmes protocoles.

Les résultats de ces mesures sont regroupés au tableau 1 ci-après.

### Exemple comparatif 2

Une composition de l'art antérieur décrite par Grispino, R.R.J. dans *The effects of luminol on serological analysis of dried blood stains Crime Laboratory Digest, 17(1), 13-23,* a été préparée. Cette composition contient 5,6 mmoles/L de luminol, 472 mmoles/L de Na₂CO₃ et 100 mmoles/L de H₂O₂, dilués dans 1 litre d'eau distillée.

Les mêmes mesures qu'à l'exemple 1 ont été effectuées, selon les mêmes protocoles.

Les résultats de ces mesures sont regroupés au tableau 1 ci-après.

### Exemple comparatif 3

Une composition contenant les mêmes concentrations de luminol, de H₂O₂ et d'agent alcalin que la composition de l'exemple comparatif 2 décrite par Grispino, R.R.J. dans *The effects of luminol on serological analysis of dried blood stains Crime Laboratory Digest, 17(1), 13-23,* a été préparée.

Cependant, dans cette composition, l'agent alcalin est K₂CO₃ et non Na₂CO₃.

Les mêmes mesures que celles décrites à l'exemple 1 ont été effectuées avec cette composition contenant en tant qu'agent alcalin K₂CO₃.

Les résultats de ces mesures sont regroupés au tableau 1 ci-après.

### Exemple comparatif 4

On a préparé une composition telle que décrite par Grodsky, M., Wright, K. & Kirk, P.L. dans *Simplified preliminary blood testing. An improved technique and comparative study of methods. Journal of the America Institute of Criminal Law and Criminalogy, 42, 95-104,* et Byrne dans les brevets U.S. 5 770 116 et 5 833 887. Cette composition contient 5,6 mmoles/L de luminol, 472 mmoles/L de Na₂CO₃ et en tant qu'agent oxydant 45,5 mmoles/L de NaBo₃.

Avec cette composition, les mêmes mesures qu'à l'exemple 1 ont été effectuées, selon les mêmes protocoles.

Les résultats de ces mesures sont regroupés au tableau 1 ci-après.

### Exemple comparatif 5

On a préparé une composition telle que décrite par Weber, K. en 1966 dans *Die Anweldung der Chemiluminescenz des Luminols in der Gerichtlichen Medizin und Toxicologie I. Der Nachweis von Blutspuren. Deutsche Zeitschrift für die gesamte Gerichtliche Medizin, 57 410-92.3.* Cette composition contient 0,4 mmoles/L de luminol, 45 mmoles/L de NaOH et 17,6 mmoles/L de H₂O₂, dilués dans un litre d'eau distillée.

Les mêmes mesures qu'à l'exemple 1 ont été effectuées, selon les mêmes protocoles.

Les résultats de ces mesures sont regroupés au tableau 1 ci-après.

### Exemple comparatif 6

On a préparé une composition contenant 0,4 mmoles/L de luminol, 45 mmoles/L de KOH et 17,6 mmoles/L de H₂O₂, dilués dans un litre d'eau distillée.

Cette composition correspond à la composition décrite par Weber, K. en 1966 dans *Die Anweldung der Chemiluminescenz des Luminols in der Gerichtlichen Medizin und Toxicologie. L Der Nachweis von Blutspuren. Deutsche Zeitschrift für die gesamte Gerichtliche Medizin, 57, 410-423,* selon l'exemple comparatif 5 mais dans laquelle la soude est remplacée par l'hydroxyde de potassium.

Les mêmes mesures qu'à l'exemple 1 ont été effectuées avec cette composition, selon les mêmes protocoles.

Les résultats de ces mesures sont regroupés au tableau 1 ci-après.

**TABLEAU 1**

| **Composition** | **pH** | **Sang dilué à 1: 1 000 000** | **sang dilué à 1:100 000** | **Sang pur frais** | **Sang pur séché** |
|---|---|---|---|---|---|
| **Exemple 1 selon l'invention** Luminol 5 mmoles/L NaOH 50 mmoles/L H₂O₂ 50 mmoles/L | 11,78 | I : 75 u.a. Durée de réaction : 4 min | I : 1106 u.a. Durée de réaction : 4 min | 1 : 66430 u.a./mm² Durée de réaction : 40 sec | I : 48405 u.a./mm² Durée de réaction : 40 sec |
| **Exemple 2 selon l'invention** Luminol 5 mmoles/L NaOH 90 mmoles/L H₂O₂ 50 mmoles/L | 12,36 | I : 394 u.a. Durée de réaction : 2 min | I : = saturation | 1 : 72043 u.a./mm² Durée de réaction : 40 sec | I : 73805 u.a./mm² Durée de réaction : 1 min |
| **Exemple 3 selon l'invention** Luminol 5 mmoles/L NaOH 25 mmoles/L H₂O₂ 50 mmoles/L | 11,12 | I : 15 u.a. Durée de réaction : 4 min | I : 123 u.a. Durée de réaction : 4 min | I : 37445 u.a./mm² Durée de réaction : 30 sec | I : 39733 u.a./mm² Durée de réaction : 30 sec |
| **Exemple 4 selon l'invention** Luminol 5 mmoles/L NaOH 40 mmoles/L H₂O₂ 50 mmoles/L | 11,5 | I : non testé Durée de réaction : non testée | I : non testé Durée de réaction : non testée | I : 55 165 u.a./mm² Durée de réaction : | I : 44930 u.a./mm² Durée de réaction : |
| **Exemple comparatif 1** Luminol 5 mmoles/L NaOH 10 mmoles/L H₂O₂ 50 mmoles/L | 10,21 | I : 4 u.a. Durée de réaction : 4 min | I : 24 u.a. Durée de réaction : 4 min | I : 19480 u.a./mm² Durée de réaction : 30 sec | I : 17970 u.a./mm² Durée de réaction : 20 sec |
| **Exemple comparatif 2** Luminol 5,6 mmoles/L Na₂CO₃ 472 mmoles/L H₂O₂ 100 mmoles/L | 10,72 | I : 96 u.a. Durée de réaction : > 15 min | I : 250 u.a. Durée de réaction : 4 min | I : 31235 u.a./mm² Durée de réaction : 50 sec | I : 30973 u.a./mm² Durée de réaction : 40 sec |
| **Exemple comparatif 3** Luminol 5,6 mmoles/L K₂CO₃ 472 mmoles/L H₂O₂ 100 mmoles/L | 10,95 | I : 30 u.a. Durée de réaction : > 15 min | I : 173 u.a. Durée de réaction : 4 min | I : 33360 u.a./mm² Durée de réaction : 40 sec | I : 26405 u.a./mm² Durée de réaction : 30 sec |
| **Exemple comparatif 4** Luminol 5,6 mmoles/L Na₂CO₃ 472 mmoles/L NaBo₃ 45,5 mmoles/L | 10,90 | I : 155 u.a. Durée de réaction : > 15 min | I : 529 u.a. Durée de réaction : 4 min | I : 31803 u.a./mm² Durée de réaction : 30 sec | I : 32175 u.a./mm² Durée de réaction : 30 sec |
| **Exemple comparatif 5** Luminol 0,4 mmoles/L NaOH 45 mmoles/L H₂O₂ 17,6 mmoles/L | 12,33 | I : 245 u.a. Durée de réaction : 8 min | I : 1542 u.a. Durée de réaction : > 15 min | I : 31210 u.a./ mm² Durée de réaction : 20 sec | I : 29853 u.a./mm² Durée de réaction : 20 sec |
| **Exemple comparatif 6** Luminol 0,4 mmoles/L KOH 45 mmoles/L H₂O₂ 17,6 mmoles/L | 11,86 | I : 255 u.a. Durée de réaction : 4 min | I : 1402 u.a. Durée de réaction : 10 min | I : 29020 u.a./mm² Durée de réaction : 20 sec | I : 27645 u.a./mm² Durée de réaction : 20 sec |

Lors de ces essais, on a constaté qu'après agitation en présence de sang, les solutions d'hydroxyde restent claires alors que les solutions contenant du carbonate mettent en évidence un dégagement gazeux.

On peut voir à partir du tableau 1 que ce sont les compositions contenant en tant qu'agent alcalin de l'hydroxyde de sodium ou de potassium plutôt que du carbonate de soude ou de potassium qui donnent les meilleurs résultats en terme d'intensité lumineuse émise.

De la même façon, on constate que lorsque pulvérisées sur du sang pur frais ou séché, ces compositions donnent la plus forte émission lumineuse.

Bien que les résultats ne soient pas reportés au tableau 1, on a aussi constaté qu'avec les compositions de l'invention ou de l'art antérieur contenant de l'hydroxyde de potassium, l'émission lumineuse se produit par un flash à retardement apparaissant lors de la dilution de la tâche de sang, ce qui a mené à la mise à l'écart de cet agent alcalin.

Les mêmes résultats ont été obtenus avec les compositions de l'invention lorsque vaporisées sur une trace de sang lavée à l'eau de javel, rincée à l'eau et essuyée.

On peut également constater à partir du tableau 1 que la composition préférée de l'invention, en terme d'émission lumineuse, à la fois sur sang frais et sur sang étalé et séché environ 30 minutes, est une composition contenant 5 mmoles/L de luminol, 90 mmoles/L de NaOH et 50 mmoles/L de H₂O₂, dilués dans de l'eau.

En effet, aussi bien sur sang frais que sur sang étalé et séché, la solution obtenue avec cette composition préférée de l'invention provoque une émission de lumière très importante qui atteint un palier lorsqu'on augmente la concentration en NaOH au-delà de 90 mmoles/L.

Cette composition est donc particulièrement adaptée pour révéler des traces de sang perdu par un gibier blessé, même dans des conditions de lumière réduite comme à la tombée du jour ou par nuit claire, en opposition à une obscurité totale.

Cependant, une telle composition a un pH supérieur à 12, ce qui peut être gênant lorsqu'on l'utilise pour détecter et localiser des traces de sang sur la scène d'un crime ou d'un accident, car à ce pH l'identification de l'ADN du sang révélé pourrait être compromise.

En effet, à un pH supérieur ou égal à 12, le sang localisé grâce à la composition de luminol ne peut faire l'objet, pour identification, d'analyses d'ADN fiables et reproductibles.

En effet, il a pu être observé que lorsque la composition du luminol a un pH supérieur ou égal à 12, l'ADN du sang "traité" avec la composition est dégradé.

Mais la composition selon l'invention contenant 5 mmoles/L de luminol, 50 mmoles/L de H₂O₂, de 25 à 50 mmoles de NaOH a quant à elle, un pH inférieur à environ 11,5, ce qui permet de réaliser des analyses d'ADN fiables et reproductibles, ce qui a été confirmé par l'Institut de Recherche Criminelle de la Gendarmerie Nationale (IRCGN).

En-dessous d'une concentration de 25 mmoles/L de NaOH, l'intensité lumineuse émise par les compositions devient relativement équivalente à celle obtenue avec les compositions de l'art antérieur.

Ainsi, pour une application à la recherche criminelle, la composition préférée selon l'invention, est une composition contenant 5 mmoles/L de luminol, 50 mmoles/L de peroxyde d'hydrogène, et de 25 à 50 mmoles/L de NaOH, dilués dans de l'eau, car ayant un pH au plus égal à environ 11,5.

Autrement dit, la concentration de la soude est celle nécessaire pour que le pH de la solution finale soit inférieur à 12. En effet, cette concentration nécessaire de soude peut varier en fonction de la nature des excipients nécessaires à la réalisation des pastilles, qui seront décrites ci-après, et qui peuvent modifier le pH de la solution, ce qui nécessitera plus ou moins de soude pour obtenir un pH final inférieur à 12.

Un autre avantage des compositions de l'invention est qu'elles peuvent être diluées dans tout milieu aqueux qui permet la solubilisation des différents composants, à l'exception de l'eau de mer et des eaux gazeuses ou gazifiées, qui inhibent la réaction.

Ainsi les compositions de l'invention ont été testées diluées dans de l'eau d'un lac, d'une rivière, d'un étang, d'une mare, d'une flaque d'eau, de l'eau distillée, de l'eau du robinet, sans perdre leur efficacité.

On l'aura compris, les compositions de l'invention sont à utiliser comme dans l'art antérieur, c'est-à-dire que leurs composants doivent être dilués dans l'eau, pour être pulvérisées à l'aide d'un pulvérisateur sur les lieux où l'on recherche des traces de sang.

Ceci signifie que les compositions sont à utiliser sous forme d'une solution.

Cette solution peut être préparée à l'avance et transportée sur les lieux où la recherche de traces de sang est à effectuer ou bien elle peut être préparée directement sur les lieux, auquel cas on transportera, dans des récipients séparés chaque composant de la composition de l'invention sous forme solide.

Comme les compositions de l'invention peuvent être diluées dans toute eau douce non gazeuse, on pourra soit prévoir d'emporter une quantité suffisante d'eau douce non gazeuse, soit trouver cette eau douce non gazeuse directement sur place.

Par conséquent, un autre objet de l'invention est un nécessaire pour la préparation d'une solution à pulvériser qui permet de détecter et de localiser des traces de sang humain ou animal, ce nécessaire étant tel que précédemment défini dans la présente description ou par les revendications qui suivent.

Pour une application à la chasse, de préférence le nécessaire selon l'invention comprend :
- dans un premier récipient au moins une dose individuelle contenant 5 mmoles du luminol ou un composé de luminol,
- dans un second récipient au moins une dose individuelle contenant 50 mmoles de peroxyde d'hydrogène, et
- dans un troisième récipient au moins une dose individuelle contenant 90 mmoles de NaOH, ou
- dans un récipient au moins une dose individuelle contenant environ 5 mmoles du luminol ou un composé de luminol en pré mélange soit avec 90 mmoles de NaOH, soit avec 50 mmoles de H₂O₂, sous forme compatible solide, et dans un second récipient au moins une dose individuelle contenant soit 50 mmoles de H₂O₂, soit 90 mmoles de soude ;
- ou enfin un pré mélange sous forme compatible de ces trois composants dans un récipient unique.

En revanche, pour une application à la recherche de traces de sang sur la scène d'un crime ou d'un accident, lorsque le sang doit ensuite subir une analyse d'ADN, de préférence, le nécessaire selon l'invention comprend :
- dans un premier récipient au moins une dose individuelle contenant 5 mmoles du luminol ou un composé de luminol,
- dans un second récipient au moins une dose individuelle contenant 50 mmoles de peroxyde d'hydrogène, et
- dans un troisième récipient au moins une dose individuelle contenant de 25 à 50 mmoles de NaOH ; ou
- dans un récipient au moins une dose individuelle contenant 5 mmoles du luminol ou un composé de luminol en pré mélange avec de 25 à 50 mmoles de NaOH ou 50 mmoles de H₂O₂, sous forme compatible solide, et
- dans un autre récipient au moins une dose individuelle contenant au moins 50 mmoles de H₂O₂, ou 25 à 50 mmoles de soude selon le pré mélange du premier récipient ; ou enfin
- un pré mélange sous forme compatible de ces trois composants dans un récipient unique.

Comme il a été dit précédemment chaque récipient peut être un récipient refermable en matière plastique ou en verre, en porcelaine etc. Il peut être par exemple un sachet avec chaque composant sous forme de poudre.

De préférence, au moins un ou chaque récipient est formé par une alvéole conformée dans au moins un blister.

Dans ce cas et dans une première variante le nécessaire contient au moins un blister comprenant trois alvéoles, avec dans chacune de ses alvéoles un composé de la composition de l'invention.

Dans ce cas, le blister contiendra dans une de ses alvéoles la dose individuelle voulue de luminol ou de composé de luminol, une autre de ses alvéoles contiendra la dose individuelle voulue de peroxyde d'hydrogène et la troisième alvéole contiendra la dose individuelle voulue de soude.

Dans une seconde variante, le nécessaire selon l'invention contiendra au moins un blister à au moins deux alvéoles, l'une de ces au moins deux alvéoles contenant la dose individuelle voulue de luminol ou de composé de luminol, en pré mélange avec la dose individuelle voulue soit de soude, soit de peroxyde d'hydrogène sous forme solide compatible, une autre de ces au moins deux alvéoles contenant la dose individuelle voulue soit de peroxyde d'hydrogène, soit de soude selon le premier pré mélange.

Dans ces deux variantes, de préférence, au moins l'une des catégories de doses individuelles de luminol ou de composé de luminol ou de mélange de ceux-ci avec la soude, de peroxyde d'hydrogène et de soude est sous la forme de pastille.

Cependant, de préférence, dans ces deux variantes chacune des doses est sous la forme de pastille.

Selon une troisième variante, le nécessaire comprend au moins un blister à au moins une alvéole qui contient une dose individuelle d'une formulation pré mélangée sous forme compatible solide des trois composants de base de l'invention. Cependant, de préférence dans cette variante, cette dose individuelle est sous forme de pastille.

En particulier, mais non limitativement, lorsque les doses individuelles sont sous la forme de pastille, celles-ci pourront de plus contenir des excipients facilitant la compression directe de la pastille afin d'éviter une granulation humide de part la présence de NaOH (lactose, cellulose, phosphate de calcium, etc.).

Elles pourront également contenir tout excipient connu de l'homme de l'art (croscarmellose, explotab, etc.) facilitant la désagrégation de la pastille dans le solvant aqueux.

Pour reconstituer la solution voulue, à l'aide, en particulier, du nécessaire selon l'invention, on diluera, dans de l'eau, ou dans un solvant aqueux, une dose individuelle de luminol ou de composé de luminol ou une dose individuelle de pré mélange de ceux-ci avec soit la soude, soit le peroxyde d'hydrogène sous forme solide compatible et, une dose individuelle soit de peroxyde d'hydrogène, soit de soude selon le premier pré mélange. Lorsque la soude ou le peroxyde d'hydrogène n'est pas dans la même dose que le luminol ou le composé de luminol une dose individuelle de soude ou de peroxyde d'hydrogène est prélevée dans les récipients du nécessaire selon l'invention. On peut prévoir aussi un pré mélange unique des trois composants de l'invention.

Pour rechercher et localiser, dans des conditions de lumière réduite, un animal blessé ou abattu, on pulvérise la composition selon l'invention, éventuellement obtenue à l'aide du nécessaire selon l'invention, sur les zones du terrain où l'on suppose que l'animal est passé, pour produire une réaction lumineuse par contact de la composition avec les traces de sang perdu par l'animal.

On procédera de la même façon pour rechercher et localiser de traces de sang humain sur la scène d'un crime ou d'un accident.

L'invention n'est nullement limitée aux modes de réalisation décrits et illustrés qui n'ont été donnés qu'à titre d'exemples illustratifs et non limitatifs.

Ainsi, bien que seul le luminol ait été cité dans les exemples qui précèdent, tous les composés de luminol pourront être utilisés dans la mesure où ils seront présents en une quantité suffisante pour fournir des mesures de luminescence équivalentes à celles obtenues avec du luminol, telle que définie dans l'invention. Des exemples de tels composés de luminol sont le diéthyl isoluminol et l'aminobutyléthyl isoluminol.

## Revendications

1. Composition pour la détection de traces de sang humain ou animal comprenant un composé de luminol, un agent oxydant et une base, dilués dans un solvant de préférence aqueux, **caractérisée en ce que** :
- le composé de luminol est présent en une quantité fournissant une concentration de 1 à 20 mmoles/L dans la composition finale,
- l'agent oxydant est du peroxyde d'hydrogène qui est présent à une concentration de 25 à 100 mmoles/L dans la composition finale,
- la base est de la soude NaOH qui est présente à une concentration comprise entre 25 mmoles/L et 500 mmoles/L dans la composition finale.

2. Composition suivant la revendication 1, **caractérisée en ce que** le pH est < 11,5.

3. Composition selon la revendication 1 ou 2, **caractérisée en ce que** le composé de luminol est choisi parmi le luminol, le diéthylisoluminol et l'aminobutyléthylisoluminol.

4. Composition selon la revendication 1, 2 ou 3, **caractérisée en ce que** la soude NaOH est présente à une concentration comprise entre 25 et 150 mmoles/L dans la composition finale.

5. Composition selon l'une quelconque des revendications 1 à 4, **caractérisée en ce que** la soude NaOH est présente à une concentration de 25 à 50 mmoles/L ou d'environ 90 mmotes/L dans la composition finale.

6. Composition selon l'une quelconque des revendications précédentes, **caractérisée en ce que** le solvant aqueux est de l'eau, mieux de l'eau non gazeuse.

7. Utilisation de la composition selon l'une quelconque des revendications précédentes pour détecter des traces de sang humain ou animal.

8. Utilisation de la composition selon la revendication 4 pour détecter des traces de sang animal sur un terrain de chasse.

9. Utilisation de la composition selon la revendication 2 ou 5 pour détecter des traces de sang humain sur la scène d'un crime ou d'un accident.

10. Nécessaire pour la préparation de la composition selon l'une quelconque des revendications 1 à 6, **caractérisé en ce qu'**il comprend :
- dans un premier récipient au moins une dose individuelle contenant un composé de luminol en une quantité fournissant de 1 à 20 mmoles ,
- dans un second récipient au moins une dose individuelle contenant de 25 à 100 mmoles de peroxyde d'hydrogène, et
- dans un troisième récipient au moins une dose individuelle contenant de 25 à 500 mmoles de soude NaOH.

11. Nécessaire selon la revendication 10, **caractérisé en ce qu'**il comprend :
- dans un premier récipient au moins une dose individuelle contenant un composé de luminol en une quantité fournissant de 1 à 10 mmoles de luminol,
- dans un second récipient au moins une dose individuelle contenant de 25 à 100 mmoles de peroxyde d'hydrogène, et
- dans un troisième récipient au moins une dose individuelle contenant de 25 à 150 mmoles de NaOH.

12. Nécessaire selon la revendication 10 ou 11, en particulier pour une utilisation sur le lieu d'un crime, **caractérisé en ce qu'**il comprend :
- dans un premier récipient au moins une dose individuelle contenant un composé de luminol en une quantité fournissant environ 5 mmoles,
- dans un second récipient au moins une dose individuelle contenant environ 50 mmoles de peroxyde d'hydrogène, et
- dans un troisième récipient au moins une dose individuelle contenant de 25 à 50 mmoles de NaOH.

13. Nécessaire selon l'une quelconque des revendications 10 à 12, **caractérisé en ce que** chaque récipient est un récipient refermable en matière plastique ou en verre.

14. Nécessaire selon l'une quelconque des revendications 10 à 13, **caractérisé en ce qu'**au moins un ou chaque récipient est formé par une alvéole conformée dans au moins un blister.

15. Nécessaire selon l'une quelconque des revendications 10 à 14, **caractérisé en ce que** l'une au moins des doses individuelles est sous la forme de pastilles.

16. Nécessaire selon l'une quelconque des revendications 10 à 15, **caractérisé en ce que** la dose individuelle contient de plus des excipients facilitant la compression directe de la pastille afin d'éviter une granulation humide de part la présence de NaOH, tels que lactose, cellulose, phosphate de calcium ; et des excipients facilitant la désagrégation de la pastille tels que croscarmellose, explotab.

17. Nécessaire selon l'une quelconque des revendications 10 à 16, **caractérisé en ce que** le composé de luminol est le luminol.

18. Nécessaire selon l'une quelconque des revendications 10 à 17, **caractérisé en ce que** les trois composants composé de luminol, soude et peroxyde d'hydrogène sont formulés en pré mélange unique dans des formulations permettant leur compatibilité sans réaction prématurée, permettant leur inclusion combinée dans un récipient unique.

19. Procédé de reconstitution de la composition selon l'une quelconque des revendications 1 à 6, **caractérisé en ce qu'**il consiste à diluer dans de l'eau une dose individuelle de composé de luminol, une dose individuelle de soude ou une dose individuelle de mélange de composé de luminol et de soude, et une dose individuelle de peroxyde d'hydrogène, prélevées dans les récipients du nécessaire selon l'une quelconque des revendications 10 à 18.

20. Procédé de recherche et de localisation, dans des conditions de lumière réduite, d'un animal blessé ou abattu, **caractérisé en ce qu'**on pulvérise la composition selon l'une quelconque des revendications 1 à 6 ou la composition obtenue selon le procédé selon la revendication 19, sur les zones du terrain où l'on soupçonne l'animal d'être passé, pour produire une réaction lumineuse par contact de la composition avec les traces de sang perdu par l'animal.

21. Procédé de recherche et de localisation de traces de sang humain sur la scène d'un crime ou d'un accident, **caractérisé en ce qu'**on pulvérise, dans des conditions de lumière réduite, la composition selon l'une quelconque des revendications 1 à 6 ou la composition obtenue par le procédé selon la revendication 19 sur la dite scène, pour produire une réaction lumineuse par contact de la composition avec les traces de sang humain.

## Claims

1. A composition for the detection of traces of human or animal blood. The aforementioned composition comprises a luminol compound, an oxidising agent and a base which are diluted in a preferably aqueous solvent. Said composition is **characterized in that** :
• the luminol compound is present in a quantity providing a concentration of between 1 and 20 mmoles/I in the end composition,
• the oxidizing agent is hydrogen peroxide which is present in a concentration of between 25 and 100 mmoles/l in the end composition,
• the base is soda, NaOH, which is present in a concentration of between 25 mmoles/I and 500 mmoles/I in the end composition.

2. Composition in accordance with claim 1, **characterised in that** the pH is < 11,5.

3. Composition in accordance with subject claim 1 or 2, **characterised in that** the luminol compound is selected from amongst luminol, diethyl isoluminol, and aminobutylethyl isoluminol.

4. Composition in accordance with subject claim 1, 2 or 3, **characterised in that** soda, NaOH, is present in a concentration of between 25 and 150 mmoles/l in the end composition.

5. Composition in accordance with any one of claims 1 to 4, **characterised in that** soda, NaOH, is present in a concentration of between 25 and 50 mmoles/l or of about 90 mmoles/l in the end composition.

6. Composition in accordance with any one of the preceding subject claims, **characterised in that** the aqueous solvent is water, and its being non-carbonated water.

7. Utilisation of the composition in accordance with any one of the preceding subject claims for the detection of traces of human or animal blood.

8. Utilisation of the composition in accordance to subject claim 4 for the detection of traces of animal blood on hunting grounds.

9. Utilisation of the composition in accordance with subject claim 2 or 5 for the detection of traces of human blood at the scene of a crime or of an accident.

10. A field kit for the preparation of the composition according to any one of the subject claims 1 to 6, **characterised in that** this kit contains:
- in a first receptacle, at least an individual dosage of luminol compound in a quantity ranging between 1 and 20 mmoles;
- in a second receptacle, at least an individual dosage containing between 25 and 100 mmoles of hydrogen peroxide, and
- in a third receptacle, at least an individual dosage containing between 25 and 500 mmoles of soda, NaOH.

11. A field kit in accordance with subject claim 9, **characterised in that** this kit contains:
- in a first receptacle, at least an individual dosage containing a luminol compound in a supply quantity of 1 to 10 mmoles of luminol,
- in a second receptacle, at least an individual dosage containing between 25 and 100 mmoles of hydrogen peroxide, and
- in a third receptacle, at least an individual dosage containing between 25 and 150 mmoles of soda, NaOH.

12. A field kit in accordance with subject claim 10 or 11, in particular for utilisation at the scene of a crime, **characterised in that** this kit contains:
- in a first receptacle, at least an individual dosage containing a luminol compound in a quantity sufficient to provide approximately 5 mmoles,
- in a second receptacle, at least an individual dosage containing approximately 50 mmoles of hydrogen peroxide, and
- in a third receptacle, at least an individual dosage containing between 25 and 50 mmoles of soda, NaOH.

13. A field kit in accordance with any one of the subject claims 10 to 12, **characterised in that** each receptacle is a resealable receptacle made of plastic material or of glass.

14. A field kit in accordance with any one of the subject claims 10 to 13, **characterised in that** at least one receptacle - or all of the receptacles - is formed by an alveolus fitted inside at least one blister pack.

15. A field kit in accordance with any one of the subject claims 10 to 14, **characterised in that** at least one of the individual dosages is in the form of a pill.

16. A field kit in accordance with any one of the subject claims 10 to 15, **characterised in that** the individual dosage further contains excipients to facilitate the direct crushing of the pill in order to avoid the formation of moist granulation due to the presence of NaOH, such as lactose, cellulose, calcium phosphate; it also contains excipients that will facilitate the disintegration of the pill, such as croscarmellose, explotab.

17. A field kit in accordance with any one of the subject claims 10 to 16, **characterised in that** the luminol compound is luminol.

18. A field kit in accordance with any one of the subject claims 10 to 17, **characterised in that** the three components luminol, soda, and hydrogen peroxide have been formulated in a single pre-mixture in formulations that allow their compatibility without generating a premature reaction, thus making it possible to enclose them jointly in one single receptacle.

19. Procedure of reconstitution of the composition in accordance with any one of the subject claims 1 to 6, **characterised in that** it consists of a dilution in water of an individual dosage of a luminol compound, an individual dosage of soda, or an individual dosage of a mixture of a luminol compound and soda, and an individual dosage of hydrogen peroxide, taken from the receptacles of the kit in accordance with any one of the subject claims 10 to 18.

20. Procedure of search and localisation of a wounded or struck down animal in conditions of reduced visibility, **characterised in that** the composition is vaporised in accordance with any one of the subject claims 1 to 6, or the composition obtained in accordance with the procedure in accordance with the subject claim 19, on the areas of the terrain where the animal is assumed to have passed, in order to produce a luminous reaction through the contact of the composition with the blood traces left behind by the hunted animal.

21. Procedure of search and localisation of traces of human blood at the scene of a crime or of an accident in conditions of reduced visibility, **characterised in that** the composition is vaporised in accordance with any one of the subject claims 1 to 6, or the composition obtained in accordance with the subject claim 19, vaporised on the mentioned scene, in order to produce a luminous reaction through the contact of the composition with the traces of human blood.

## Patentansprüche

1. Zusammensetzung für den Nachweis von menschlichen oder tierischen Blutspuren, welche eine Luminolverbindung, ein Oxidationsmittel und eine Base umfasst, die in einem vorzugsweise wässrigen Lösemittel verdünnt sind, **dadurch gekennzeichnet, dass**:
- die Luminolverbindung in einer Menge vorhanden ist, die in der gebrauchsfertigen Zusammensetzung eine Konzentration zwischen 1 und 20 mmol/L liefert,
- es sich bei dem Oxidationsmittel um Wasserstoffperoxid handelt, das in der gebrauchsfertigen Zusammensetzung in einer Konzentration zwischen 25 und 100 mmol/L vorhanden ist,
- es sich bei der Base um Ätznatron NaOH handelt, das in der gebrauchsfertigen Zusammensetzung in einer Konzentration zwischen 25 und 500 mmol/L vorhanden ist.

2. Zusammensetzung gemäß Anspruch 1, **dadurch gekennzeichnet, dass** der pH-Wert < 11,5 ist.

3. Zusammensetzung gemäß Anspruch 1 oder 2, **dadurch gekennzeichnet, dass** die Luminolverbindung aus Luminol, Diethylisoluminol und Aminobutylethylisoluminol gewählt ist.

4. Zusammensetzung gemäß den Ansprüchen 1, 2 oder 3, **dadurch gekennzeichnet, dass** das Ätznatron NaOH in der gebrauchsfertigen Zusammensetzung in einer Konzentration zwischen 25 und 150 mmol/L vorhanden ist.

5. Zusammensetzung gemäß den Ansprüchen 1 bis 4, **dadurch gekennzeichnet, dass** das Ätznatron NaOH in der gebrauchsfertigen Zusammensetzung in einer Konzentration zwischen 25 und 50 mmol/L, oder von ungefähr 90 mmol/L, vorhanden ist.

6. Zusammensetzung gemäß einem beliebigen der vorhergehenden Ansprüche, **dadurch gekennzeichnet, dass** es sich bei dem wässrigen Lösemittel um Wasser, besser um Wasser, in dem keine Gase gelöst sind, handelt.

7. Verwendung der Zusammensetzung gemäß einem beliebigen der vorhergehenden Ansprüche zum Nachweis von menschlichen oder tierischen Blutspuren.

8. Verwendung der Zusammensetzung gemäß Anspruch 4 zum Nachweis von tierischen Blutspuren in einem Jagdrevier.

9. Verwendung der Zusammensetzung gemäß den Ansprüchen 2 oder 5 zum Nachweis von menschlichen Blutspuren am Tatort eines Verbrechens oder an einem Unfallort.

10. Anwendungskit für die Herstellung der Zusammensetzung gemäß einem beliebigen der Ansprüche 1 bis 6, **dadurch gekennzeichnet, dass** es Folgendes umfasst:
- in einem ersten Behälter mindestens eine Einzeldosis, die eine Luminolverbindung in einer Menge enthält, die 1 bis 20 mmol liefert,
- in einem zweiten Behälter mindestens eine Einzeldosis, die 25 bis 100 mmol an Wasserstoffperoxid enthält, und
- in einem dritten Behälter mindestens eine Einzeldosis, die 25 bis 500 mmol an Ätznatron NaOH enthält.

11. Anwendungskit gemäß Anspruch 10, **dadurch gekennzeichnet, dass** es Folgendes umfasst:
- in einem ersten Behälter mindestens eine Einzeldosis, die eine Luminolverbindung in einer Menge enthält, die 1 bis 10 mmol an Luminol liefert,
- in einem zweiten Behälter mindestens eine Einzeldosis, die 25 bis 100 mmol an Wasserstoffperoxid enthält, und
- in einem dritten Behälter mindestens eine Einzeldosis, die 25 bis 150 mmol an NaOH enthält.

12. Anwendungskit gemäß den Ansprüchen 10 oder 11, insbesondere zur Verwendung am Tatort eines Verbrechens, **dadurch gekennzeichnet, dass** es Folgendes umfasst:
- in einem ersten Behälter mindestens eine Einzeldosis, die eine Luminolverbindung in einer Menge enthält, die ungefähr 5 mmol liefert,
- in einem zweiten Behälter mindestens eine Einzeldosis, die ungefähr 50 mmol an Wasserstoffperoxid enthält, und
- in einem dritten Behälter mindestens eine Einzeldosis, die 25 bis 50 mmol an NaOH enthält.

13. Anwendungskit gemäß einem beliebigen der Ansprüche 10 bis 12, **dadurch gekennzeichnet, dass** es sich bei sämtlichen der jeweiligen Behälter um wiederverschließbare Behälter aus Kunststoff oder Glas handelt.

14. Anwendungskit gemäß einem beliebigen der Ansprüche 10 bis 13, **dadurch gekennzeichnet, dass** mindestens einer oder sämtliche der Behälter durch jeweils eine Kavität gebildet werden, die von mindestens einem Blister umschlossen wird.

15. Anwendungskit gemäß einem beliebigen der Ansprüche 10 bis 14, **dadurch gekennzeichnet, dass** mindestens eine der Einzeldosen in Tablettenform vorliegt.

16. Anwendungskit gemäß einem beliebigen der Ansprüche 10 bis 15, **dadurch gekennzeichnet, dass** die Einzeldosis zusätzlich Trägerstoffe wie etwa Lactose, Cellulose oder Calciumphosphat enthält, welche die direkte Pressung der Tablette erleichtern, um ein Verklumpen auf der Gegenwart von NaOH zu verhindern; sowie Trägerstoffe, welche das Auflösen der Tablette erleichtern, wie etwa Croscarmellose oder Explotab.

17. Anwendungskit gemäß einem beliebigen der Ansprüche 10 bis 16, **dadurch gekennzeichnet, dass** es sich bei der Luminolverbindung um Luminol handelt.

18. Anwendungskit gemäß einem beliebigen der Ansprüche 10 bis 17, **dadurch gekennzeichnet, dass** die drei Bestandteile Luminolverbindung, Ätznatron und Wasserstoffperoxid in einer einzigen Vormischung vorliegen, wobei die jeweiligen Formulierungen für eine Verträglichkeit ohne vorzeitige Reaktion sorgen, wodurch es möglich wird, sie gemeinsam in einem einzigen Behälter vorzuhalten.

19. Verfahren zur Herstellung der Zusammensetzung gemäß einem beliebigen der Ansprüche 1 bis 6, **dadurch gekennzeichnet, dass** es darin besteht, eine Einzeldosis einer Luminolverbindung, eine Einzeldosis an Ätznatron oder eine Einzeldosis der Mischung aus Luminolverbindung und Ätznatron sowie eine Einzeldosis an Wasserstoffperoxid in Wasser zu verdünnen, wobei die Dosen aus den Behältern des Anwendungskits gemäß einem beliebigen der Ansprüche 10 bis 18 entnommen werden.

20. Verfahren zur Suche oder Lokalisierung eines verletzten oder erlegten Tieres bei vermindertem Lichteinfall, **dadurch gekennzeichnet, dass** die Zusammensetzung gemäß einem beliebigen der Ansprüche 1 bis 6 oder die Zusammensetzung, die gemäß dem Verfahren nach Anspruch 19 erhalten wird, in den Bereichen des Reviers, welche das Tier vermutlich durchquert hat, versprüht wird, sodass bei Kontakt der Zusammensetzung mit den Blutspuren, die das Tier hinterlassen hat, eine Leuchtreaktion stattfindet.

21. Verfahren zur Suche oder Lokalisierung von menschlichen Blutspuren am Tatort eines Verbrechens oder an einem Unfallort, **dadurch gekennzeichnet, dass** die Zusammensetzung gemäß einem beliebigen der Ansprüche 1 bis 6 oder die Zusammensetzung, die gemäß dem Verfahren nach Anspruch 19 erhalten wird, bei vermindertem Lichteinfall an diesem Ort versprüht wird, sodass bei Kontakt der Zusammensetzung mit den menschlichen Blutspuren eine Leuchtreaktion stattfindet.
